# FASCICULE DE BREVET EUROPEEN

(11) **EP 1 335 753 B1**
(45) Date de publication et mention de la délivrance du brevet: **23.08.2006**
(21) Numéro de dépôt: 01996396.6
(22) Date de dépôt: 16.11.2001
(51) Int. Cl.: A61L 2/26, A61L 2/08

(54) **EMBALLAGE POUR PRODUITS STERILES**
VERPACKUNG FÜR STERILE PRODUKTE
PACKAGE FOR STERILE PRODUCTS

(30) Priorité: 20.11.2000 FR 0014975
(43) Date de publication de la demande: 20.08.2003
(73) Titulaire: Becton Dickinson France, 38800 Le Pont de Claix (FR)
(72) Inventeur: PORRET, Jean-Yves, F-38610 Gières (FR); JANSEN, Hubert, D-35041 Marburg-Michelbach (FR)
(74) Mandataire: Brédeville, Odile Marie
(86) Numéro de dépôt international: PCT/FR2001/003613
(87) Numéro de publication internationale: WO 2002/040064

(56) Documents cités:
- WO-A-91/11204
- DE-A- 2 439 900
- US-A- 5 496 302
- US-A- 6 085 492

## Description

La présente invention se rapporte au domaine des emballages stériles/stérilisés et plus particulièrement aux emballages destinés à être utilisés pour transporter des produits stérilisés ou destinés à être stérilisés.

Les conditions de stérilité dans lesquelles doivent se dérouler certaines étapes de manipulation ou de transport des produits ou ustensiles destinés à un usage médical sont très contraignantes, et en particulier dans l'industrie pharmaceutique. Il est donc d'une grande importance de réaliser des emballages compatibles avec de telles exigences.

Dans la suite de la description, il sera fait mention d'un matériau sélectivement étanche qu'il convient de définir.

Par l'expression "sélectivement étanche" telle qu'utilisée dans la présente description, ainsi que dans les revendications, on entend que le matériau est conçu, en termes de structure, de manière à contrôler tout échange de l'intérieur de l'emballage avec son environnement extérieur. Ceci signifie entre autres que l'emballage est étanche, individuellement ou en combinaison à la contamination par des microorganismes, bactéries et/ou un matériau biologiquement actif, susceptibles de venir en contact avec l'emballage lors de sa manipulation, tout en restant perméable à un gaz de stérilisation ou de décontamination par exemple du type ETO (oxyde d'éthylène).

II sera également fait mention d'un "matériau formant écran vis à vis d'un rayonnement électronique", lequel doit être compris comme un matériau susceptible de réfléchir et/ou d'absorber partiellement ou en totalité l'énergie cinétique des électrons issus d'un faisceau électronique, et par conséquent de ralentir voire d'empêcher ces derniers de traverser ledit matériau.

Par "matériau plastique", il convient d'entendre tout matériau choisi dans les familles de polymères tels que les styréniques, acryliques, polysulfones, polycarbonates, polyesters, polyoléfines, etc., en incluant les copolymères, combinaisons et alliages de polymères.

On connaît des emballages pour produits stériles ou destinés à être stérilisés par un gaz du type ETO, comportant une boîte en matière plastique et une feuille de couverture en matériau sélectivement étanche, collée sur la boîte de manière à sceller cette dernière.

Il est également connu de réaliser un emballage de produits destinés à être stérilisés par un gaz, par exemple du type ETO, par un procédé consistant à utiliser une boîte en matière plastique et une feuille de couverture en matériau sélectivement étanche et à sceller de manière étanche la boîte en fixant le bord périphérique de la feuille de couverture sur la boîte.

Certains emballages comme ceux utilisés pour transporter des seringues avant leur remplissage par un produit actif ou médicament, sont actuellement transportés dans des boîtiers en matière plastique, par exemple du polystyrène, recouverts d'une feuille de couverture réalisée avec un matériau sélectivement étanche.

Ce dernier est par exemple une feuille à base de filaments de PEHD (polyéthylène haute densité) ou autre polymère, liés notamment par l'intermédiaire de chaleur et de pression. Un tel produit est par exemple commercialisé sous la marque TYVEK®. Des produits destinés à être stérilisés sont disposés à l'intérieur d'un boîtier, lequel est ensuite scellé par l'intermédiaire de la feuille sélectivement étanche. Le gaz de stérilisation, par exemple du type ETO, pénètre ensuite dans le boîtier à travers la feuille de matériau sélectivement étanche. La boîte contenant les produits stérilisés est ensuite disposée dans un sac de protection pour transporter ladite boite. A titre d'exemple, une telle boîte ou emballage peut contenir des seringues destinées à être remplies par un médicament dans une salle stérile ou à environnement contrôlé. Il est également possible de disposer au préalable l'emballage dans un sac de protection pourvu d'une fenêtre obturée par un matériau perméable au gaz de stérilisation, et de procéder ensuite à ladite stérilisation.

Avant le remplissage desdites seringues, il est nécessaire d'ouvrir le sac de protection et de décontaminer l'emballage éventuellement contaminé avant son introduction par exemple dans une salle stérile. Une telle décontamination peut être obtenue à l'aide d'une irradiation multidirectionnelle par faisceau d'électrons développant une énergie suffisante pour présenter après la traversée de la feuille de couverture, une dose d'irradiation par exemple de 25 kGy. Cela permet de considérer que le matériau sélectivement étanche, a été décontaminé dans toute son épaisseur, et en particulier la zone scellée, à l'interface de la boîte et dudit matériau. Pour le reste, le couple densité-épaisseur de l'emballage est tel qu'il arrête ces électrons.

Ce type de décontamination n'est cependant pas adapté pour tout type de produits transportés dans l'emballage. En effet, le faisceau d'électrons traversant la feuille du matériau sélectivement étanche risque d'une part de modifier ou d'altérer le matériau constitutif des seringues ou produits disposés dans la boite, par exemple du verre, et d'autre part de générer avec l'oxygène de l'air contenu dans ladite boîte de l'ozone qui risque d'une part d'altérer les produits actifs venant remplir les seringues, et/ou par exemple les éléments en caoutchouc présents dans le boîtier comme par exemple les capuchons des aiguilles montés sur les seringues, et d'autre part de polluer l'atmosphère.

Le document US-A-5496302 décrit un emballage rempli par un liquide médical et sélectivement étanche lors de la stérilisation d'an dispositif par rayonnement d'électrons auquel il est connecté, l'emballage est protégé par un écran.

L'objectif de la présente invention est de fournir un emballage pour des produits ou ustensiles destinés à être stérilisés, permettant d'utiliser un faisceau d'électrons pour réaliser la décontamination biologique de cet emballage, avant son introduction dans une salle stérile ou à environnement contrôlé, et ce, sans que les produits ou ustensiles situés à l'intérieur de l'emballage ne soient altérés.

L'objectif de la présente invention est également de fournir un procédé pour la réalisation d'un emballage pour des produits ou ustensiles destinés à être stérilisés, ledit emballage étant compatible avec une stérilisation par exemple par un gaz du type ETO d'une part, et avec une décontamination à l'aide d'un faisceau d'électrons d'autre part.

Selon l'invention, l'emballage comprend un écran vis à vis d'un rayonnement d'électrons, placé le long de la feuille de couverture du côté de l'intérieur de la boîte et dimensionné de manière à s'étendre au-dessus des produits à stériliser et à délimiter sur la feuille de couverture une zone périphérique de fixation de cette feuille de couverture sur la boîte.

Selon l'invention, le procédé consiste à :
- utiliser un écran vis-à-vis d'un rayonnement électronique,
- choisir la forme et les dimensions de cet écran de telle sorte que cet écran s'étende, lorsqu'il est dans la boîte, au-dessus des produits à stériliser et délimite sur la feuille de couverture une zone périphérique de fixation de cette feuille de couverture sur la boîte.

L'écran peut être rapporté sur la feuille de couverture notamment par collage ou soudure, ou peut être simplement disposé sur les produits à l'intérieur de la boîte, préalablement au scellage de celle-ci. L'écran peut également être formé par au moins deux feuilles ou couches, au moins une desdites feuilles ou couches étant alors rapportée sur la feuille de couverture tandis qu'au moins une autre desdites feuilles ou couches est disposée à l'intérieur de la boîte, préalablement au scellage de celle-ci, soit sur les produits, soit sur des appuis prévus à cet effet, soit sur une pièce de positionnement des produits placée dans cette boîte.

L'écran peut comprendre une feuille métallisée ou métallique, un matériau plastique, un matériau à base de fibres naturelles, par exemple végétales, ou au moins deux feuilles complémentaires de matériau sélectivement étanche.

Le matériau sélectivement étanche peut être un matériau à base de filaments de PEHD ou autre polymère, liés entre autres par l'intermédiaire de chaleur et de pression, ou un matériau à base de fibres végétales, par exemple du papier.

D'autres caractéristiques et avantages ressortiront également de la description détaillée figurant ci-après, donnée à titre d'exemple, par référence au dessin annexé, dans lequel :
la figure 1 est une vue en coupe d'un emballage conforme à l'invention ;
la figure 2 est une vue similaire à la figure 1 d'un emballage selon une variante d'exécution ;
la figure 3 est une vue similaire à la figure 1 d'un emballage selon une autre variante d'exécution ;
la figure 4 représente un autre exemple d'emballage conforme à l'invention ;
la figure 5 représente un exemple supplémentaire d'emballage conforme à l'invention ;
la figure 6 est une vue de l'emballage de la figure 4 en coupe selon la ligne VI-VI, et
la figure 7 représente un autre exemple d'emballage conforme à l'invention.

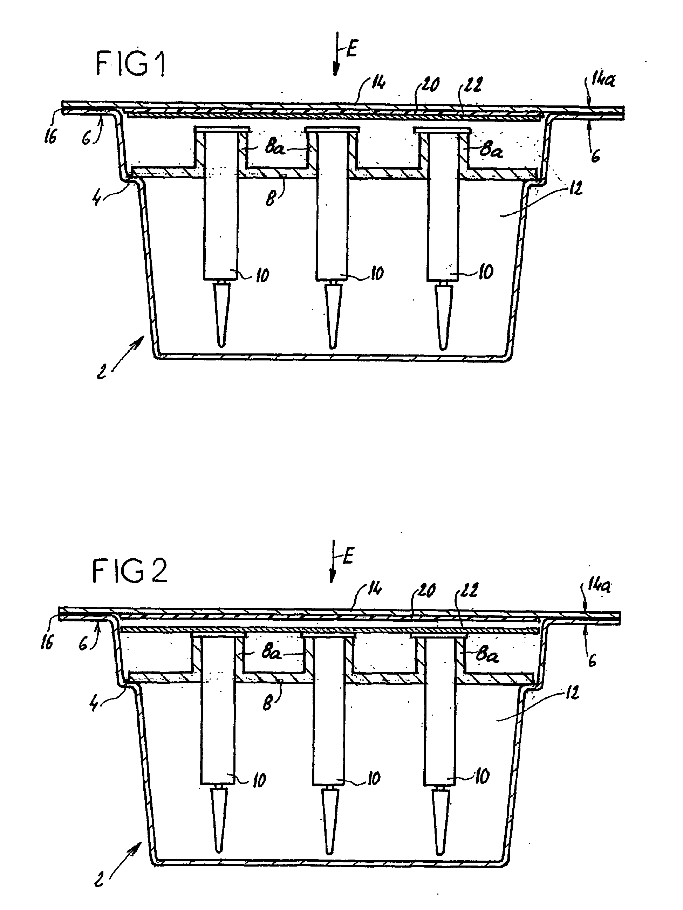

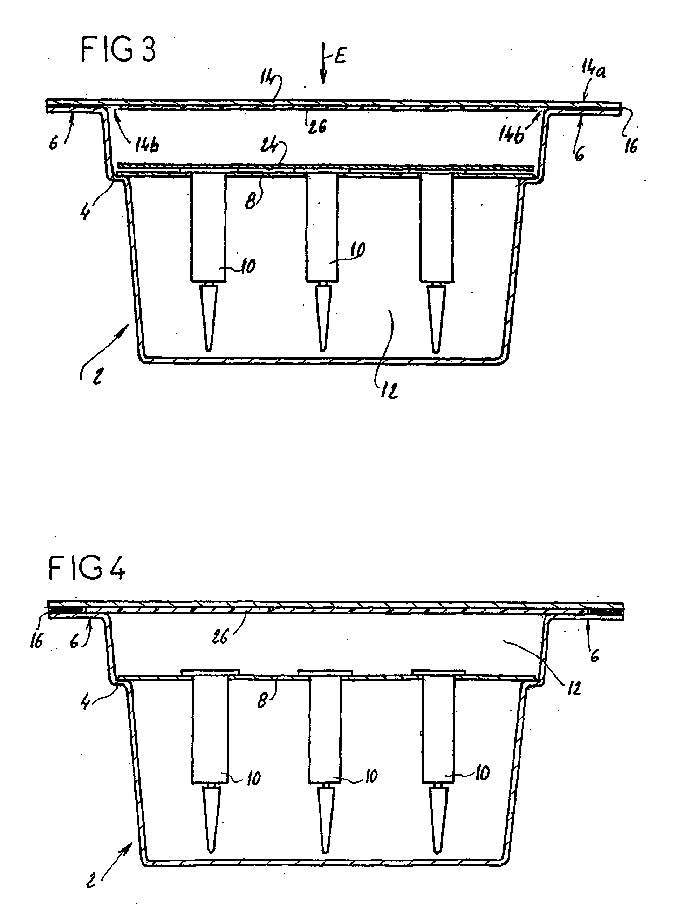

L'emballage conforme à l'invention, représenté aux figures 1 et 2, comprend une boîte 2 en matériau plastique, par exemple du polystyrène, présentant un épaulement périphérique interne 4 et un rebord périphérique externe 6 s'étendant sensiblement horizontalement à l'extrémité supérieure de la boîte. L'épaulement 4, dont l'éloignement par rapport au fond de la boîte 2 peut être choisi, permet d'y reposer un support 8. Ce support 8, qui peut comprendre des cheminées de positionnement 8a des seringues 10, comme montré sur la figure 1 ou 2, ou peut être constitué par une plaque perforée, comme montré sur les figures 3 à 7, permet de maintenir des seringues 10.

Le volume intérieur de l'emballage est désigné par la référence 12.

L'emballage comprend également une feuille de couverture 14 réalisée en un matériau sélectivement étanche, et fixée par sa zone périphérique 14a, sur le rebord périphérique 6 de la boîte 2. Une couche de colle 16 réalisant l'interface entre la feuille de couverture 14 et le rebord périphérique 6 permet de sceller de façon étanche la boîte 2 conformément à l'invention. Selon un autre mode de réalisation, la couche de colle 16 peut être remplacée par un autre moyen de liaison étanche telle qu'une soudure.

Dans l'exemple de réalisation représenté à la figure 1, l'emballage comprend également deux feuilles complémentaires 20, 22 rapportées sur la feuille de couverture 14, du côté de l'intérieur de la boîte 2, et en un matériau sélectivement étanche. La forme et les dimensions de ces feuilles complémentaires 20, 22 sont telles qu'elles permettent de délimiter sur ladite feuille de couverture 14 la zone périphérique 14a de fixation à la boîte 2. Cette zone périphérique 14a présente ainsi une épaisseur correspondant à celle de la feuille de couverture 14, tandis que la zone centrale de la feuille de couverture 14 présente une épaisseur de couverture correspondant à celle d'un assemblage de trois feuilles d'un matériau sélectivement étanche. Cet assemblage est obtenu par exemple par collage.

Un tel emballage présente l'avantage que le moyen de couverture ainsi réalisé constitué de la feuille de couverture 14 et des feuilles complémentaires 20, 22, reste entièrement perméable au gaz de stérilisation ETO. En revanche, l'épaisseur obtenue dans sa zone centrale, correspondant à un assemblage de trois feuilles sélectivement étanches, permet d'absorber substantiellement l'énergie des électrons issus d'un faisceau d'électrons utilisé pour la décontamination, schématisé par la flèche "E" sur les figures et ajusté de manière à assurer une dose d'irradiation par exemple de 25 kGy sous la feuille de couverture 14. La flèche "E" schématise le rayonnement électronique en direction de la feuille de couverture 14, mais l'irradiation de l'emballage conforme à l'invention est multidirectionnelle et vient de toutes les directions de manière à atteindre chaque côté dudit emballage. Le matériau constitutif de la boîte 2, présente un couple densité-épaisseur tel, qu'il permet pratiquement d'empêcher les électrons de ce faisceau de décontamination, de le traverser. Cette dose est nécessaire pour obtenir une décontamination à travers la feuille de couverture 14 et ce en particulier jusqu'à la couche de colle 16 réalisant l'interface entre ladite feuille de couverture 14 et la boîte 2. Le faisceau d'électrons permet avantageusement de décontaminer le matériau sélectivement étanche jusqu'à l'extrémité périphérique (bord extrême) de ce dernier. Les feuilles complémentaires 20, 22 permettent d'absorber suffisamment l'énergie des électrons pouvant traverser la feuille de couverture 14, de manière à ne pas altérer ou de ne pas risquer d'altérer les produits à l'intérieur de l'emballage d'une part, et à ne pas générer de l'ozone dans le volume intérieur 12 d'autre part.

Lorsqu'on utilise en tant qu'écran, des feuilles telles que du TYVEK®, les feuilles complémentaires 20, 22, sont par exemple au nombre de deux.

Il est également possible de recouvrir les seringues 10 disposées dans la boîte 2, d'une ou plusieurs feuilles de protection 24, par exemple en matériau sélectivement étanche.

Dans la variante représentée sur la figure 2, la feuille 22 n'est pas solidarisée à la feuille 20 mais repose sur les seringues 10.

Sur l'emballage selon la figure 3, l'écran est constitué d'une feuille métallique 26, métallisée ou en matière plastique. Une feuille d'aluminium ou d'inox, peut ainsi être rapportée sur la feuille de couverture 14.

La feuille de protection 24 peut également être utilisée dans cet exemple de réalisation.

Selon un autre mode de réalisation de l'emballage conforme à l'invention, non représenté aux figures, l'écran peut être constitué par la feuille de protection 24 elle-même. L'écran est ainsi disposé sur les seringues 10 à l'intérieur de la boîte 2, préalablement au scellage de celle-ci, et est libre dans une partie du volume intérieur 12 délimité par la feuille de couverture 14 et l'épaulement 4. La feuille de protection 24 peut être réalisée avec les mêmes matériaux que ceux utilisés pour réaliser la feuille formant écran 26, ou avec deux feuilles de matériau sélectivement étanche. Un tel emballage est très avantageux lorsque la boîte 2, scellée par la feuille de couverture 14, est retournée pendant l'opération de décontamination. Après le retournement, l'écran repose sur le côté intérieur de la feuille de couverture 14 et limite ou empêche ainsi la pénétration d'électrons à l'intérieur du volume interne 12 et limite ou empêche par conséquent la création d'ozone.

D'autres matériaux connus et susceptibles d'absorber et/ou de réfléchir l'énergie cinétique des électrons émis par un faisceau de décontamination peuvent également être utilisés pour réaliser un emballage conforme à l'invention. Des fibres d'origine naturelle, par exemple végétales (cellulose, papier), peuvent ainsi être utilisées pour réaliser l'écran. De façon générale, le matériau constitutif de l'écran présente des caractéristiques de densité et d'épaisseur, lui permettant d'absorber substantiellement une dose d'irradiation électronique de 25 kGy. L'énergie des électrons susceptibles de traverser l'écran est pratiquement réduite à zéro.

La figure 4 montre ainsi un exemple de réalisation dans lequel la feuille formant écran 26 est constituée d'un matériau sélectivement étanche et dont le rapport densité/épaisseur lui permet d'absorber la dose d'irradiation électronique précitée. Les dimensions et les formes de la feuille formant écran 26 sont choisies pour lui permettre de s'appuyer avec sa périphérie sur une partie du rebord périphérique 6. La feuille formant écran 26 est par exemple fixée par tous moyens sur la feuille de couverture 14.

Dans l'exemple de la figure 3, la feuille formant écran 26 présente des dimensions et une forme lui permettant de ménager une fente périphérique 14b entre elle et la boîte 2, facilitant ainsi la pénétration du gaz de stérilisation dans le volume interne 12.

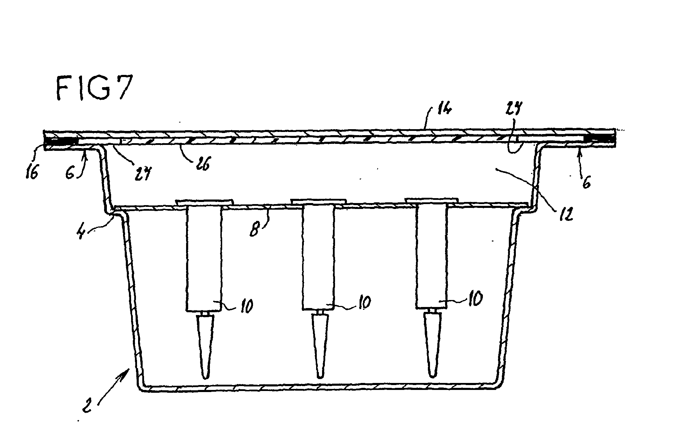

Selon un mode de réalisation de l'emballage conforme à l'invention, il est également possible d'utiliser une feuille formant écran 26, réalisée en matériau étanche, comme représenté à la figure 7. La feuille formant écran 26 repose alors sur le rebord périphérique 6 et présente des ouvertures 27 distribuées de façon discrète sur sa périphérie. Les ouvertures 27 permettent ainsi au gaz de stérilisation de pénétrer dans le volume interne 12, le rayonnement électronique susceptible de passer à travers les ouvertures 27 de dimensions relativement faibles n'ayant qu'une incidence minime sur les produits disposés à l'intérieur de la boîte 2.

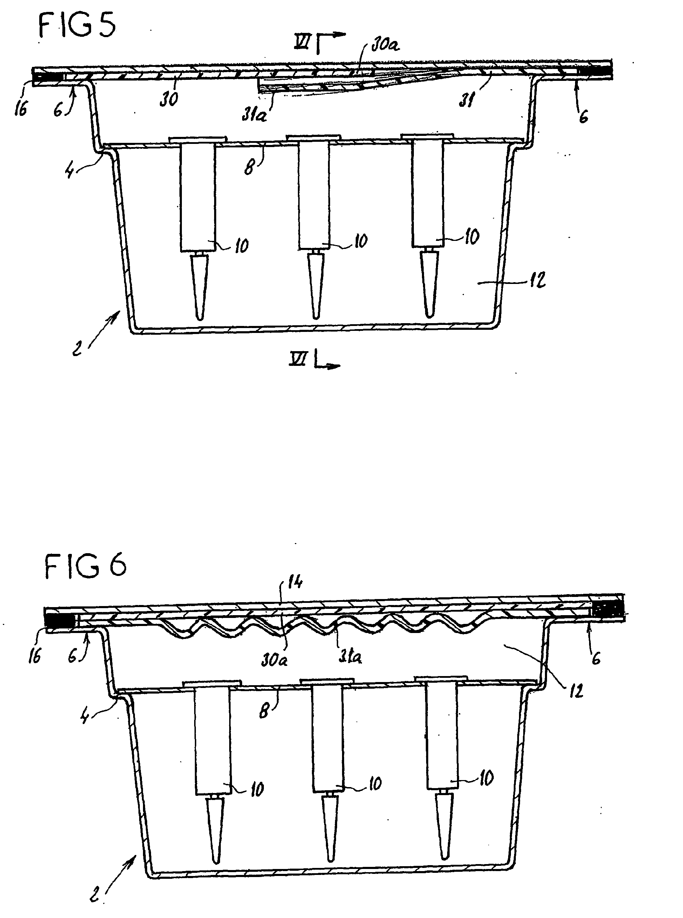

L'exemple de réalisation représenté aux figures 5 et 6 comprend deux demi-feuilles 30, 31 dont les bords externes reposent sur le rebord périphérique 6 et dont les bords internes 30a, 31a se chevauchent. L'une des demi-feuilles 31 a présente une forme ondulée de manière à réaliser des passages pour le gaz de stérilisation, en particulier lorsque le matériau constitutif des deux demi-feuilles 30,31 est étanche. Chacune des demi-feuilles 30,31 constitue un écran au rayonnement électronique. Dans la périphérie extérieure de la zone de chevauchement, on obtient une surépaisseur locale entre le rebord périphérique 6 et la feuille de couverture 14, compensée par exemple avec une couche de colle 16, localement plus épaisse. Ceci n'est pas gênant dans la mesure où le rayonnement électronique de décontamination traversant la feuille de couverture 14 est susceptible d'atteindre une éventuelle irrégularité dans ladite couche de colle 16.

Toute autre association ou transposition d'éléments techniques d'un exemple de réalisation à un autre peut être envisagé, à condition de constituer un ensemble de couverture sélectivement étanche et susceptible d'absorber une dose d'irradiation électronique, par exemple de 25 kGy.

La présente invention concerne aussi un procédé de réalisation d'un emballage de produits destinés à être stérilisés par un gaz, par exemple du type ETO. Le procédé consiste à utiliser la boîte 2 en matière plastique et la feuille de couverture 14 en matériau sélectivement étanche, et à sceller ladite boîte 2 en fixant le bord périphérique 14a de la feuille de couverture 14 sur la boîte 2.

Selon l'invention, le procédé consiste à choisir la forme et les dimensions de ce matériau pour délimiter en le rapportant sur la feuille de couverture 14, d'une part un bord périphérique 14a dont l'épaisseur est celle de ladite feuille de couverture 14, et d'autre part une zone centrale dont l'épaisseur correspond à celle d'un assemblage de la feuille de couverture 14 et de l'écran.

La réalisation d'un tel moyen de couverture pour sceller la boîte 2 est particulièrement avantageuse dans la mesure ou l'on conserve les propriétés de la feuille de couverture 14 en matériau sélectivement étanche, malgré l'adjonction d'un écran vis-à-vis du rayonnement électronique. Cet avantage est particulièrement intéressant lorsqu'on utilise deux feuilles complémentaires sélectivement étanches pour réaliser l'écran.

Selon une variante d'exécution conforme à l'invention, l'écran peut être rapporté et fixé sur la feuille de couverture 14 par soudure, thermique, haute fréquence, ultrasons ou vibrations ou autres, de même que par gaufrage ou à l'aide de rivets.

Il en est de même pour l'assemblage de la feuille de couverture 14 et de la ou des feuilles complémentaires.

Selon un mode de réalisation de l'emballage conforme à l'invention, la feuille de couverture 14 peut être réalisé avec une membrane micro perforée.

Selon une variante d'exécution du procédé conforme à l'invention, il est possible de disposer l'écran préalablement au scellage de la boîte 2, sur les produits à l'intérieur de la boîte 2.

## Revendications

1. Emballage pour produits stériles ou destinés à être stérilisés par un gaz, par exemple un gaz du type ETO, comportant une boîte (2) en matière plastique et une feuille de couverture (14) en matériau sélectivement étanche, étanche à la contamination par des microorganismes, bactéries et/ou un matériau biologiquement actif, tout en restant perméable à un gaz de stérilisation, fixée sur la boîte (2) de manière à sceller cette dernière de façon étanche ;
l'emballage comprenant un écran (20, 22 ; 24, 26 ; 26) vis-à-vis d'un rayonnement d'électrons (E), placé le long de la feuille de couverture (14) du côté de l'intérieur de la boîte (2) et dimensionné de manière à s'étendre au-dessus des produits (10) à stériliser et à délimiter sur la feuille de couverture (14) une zone périphérique (14a) de fixation de cette feuille de couverture (14) sur la boîte (2).

2. Emballage selon la revendication 1, **caractérisé en ce que** l'écran (20, 22 ; 26) est rapporté sur la feuille de couverture (14), notamment par collage ou soudure.

3. Emballage selon la revendication 1, **caractérisé en ce que** l'écran (24) est disposé sur les produits (10) à l'intérieur de la boîte (2), préalablement au scellage de celle-ci.

4. Emballage selon la revendication 1, **caractérisé en ce que** l'écran (20, 22 ; 24, 26) est formé par au moins deux feuilles ou couches et **en ce qu'**au moins une desdites feuilles ou couches (20 ; 26) est rapportée sur la feuille de couverture (14), tandis qu'au moins une autre desdites feuilles ou couches (22 ; 24) est disposée à l'intérieur de la boîte (2), préalablement au scellage de celle-ci, soit sur les produits (10), soit sur des appuis prévus à cet effet, soit sur une pièce de positionnement des produits placée dans cette boîte.

5. Emballage selon l'une des revendications 1 à 4, **caractérisé en ce que** l'écran (24 ; 26) comprend une feuille métallisée ou métallique (26).

6. Emballage selon l'une des revendications 1 à 4, **caractérisé en ce que** l'écran comprend un matériau plastique.

7. Emballage selon l'une des revendications 1 à 4, **caractérisé en ce que** l'écran comprend un matériau à base de fibres naturelles, par exemple végétales.

8. Emballage selon l'une des revendications 1 à 4, **caractérisé en ce que** l'écran comporte au moins deux feuilles complémentaires (20, 22) de matériau sélectivement étanche.

9. Emballage selon l'une quelconque des revendications 1 à 8, **caractérisé en ce que** le matériau sélectivement étanche est un matériau à base de filaments de PEHD ou autre polymère, liés entre autres par l'intermédiaire de chaleur et de pression.

10. Emballage selon l'une quelconque des revendications 1 à 8, **caractérisé en ce que** le matériau sélectivement étanche est un matériau à base de fibres végétales, par exemple du papier.

11. Procédé pour la réalisation d'un emballage selon l'une des revendications 1 à 10, consistant à :
- utiliser une boîte (2) en matière plastique et une feuille de couverture (14) en matériau sélectivement étanche étanche à la contamination par des microorganismes, bactéries et/ou un matériau biologiquement actif, tout en restant perméable à un gaz de stérilisation;
- sceller de manière étanche la boîte (2) en fixant la zone périphérique (14a) de la feuille de couverture (14) sur la boîte (2) ;
- utiliser un écran (20, 22 ; 26 , 24 ; 26) vis-à-vis d'un rayonnement électronique (E),
- choisir la forme et les dimensions de cet écran (20, 22 ; 26, 24 ; 26) de telle sorte que cet écran (20, 22 ; 26, 24 ; 26) s'étende, lorsqu'il est dans la boîte (2), au-dessus des produits (10) à stériliser et délimite sur la feuille de couverture (14) ladite zone périphérique (14a).

12. Procédé selon la revendication 11, **caractérisé en ce qu'**il comprend l'étape consistant à rapporter l'écran (20, 22 ; 26) sur la feuille de couverture (14), notamment par collage ou soudure.

13. Procédé selon la revendication 11, **caractérisé en ce qu'**il comprend l'étape consistant à disposer l'écran (24) sur les produits (10) à l'intérieur de la boîte (2), préalablement au scellage de celle-ci.

14. Procédé selon la revendication 11, **caractérisé en ce qu'**il comprend les étapes consistant :
- à utiliser un écran (20, 22 ; 24, 26) formé par au moins deux feuilles ou couches et
- à rapporter au moins une desdites feuilles ou couches (20 ; 26) sur la feuille de couverture (14) et à disposer au moins une autre desdites feuilles ou couches (22 ; 24) à l'intérieur de la boîte (2), préalablement au scellage de celle-ci, soit sur les produits (10), soit sur des appuis prévus à cet effet, soit sur une pièce de positionnement des produits placée dans cette boîte.

## Claims

1. Packaging for sterile products or products intended to be sterilized by a gas, for example a gas of the ETO type, comprising a tub (2) made of plastic and a cover sheet (14) made of a selectively impervious material, impervious to contamination by micro-organisms, bacteria and/or a biologically active material while at the same time remaining permeable to a sterilization gas, fixed to the tub (2) so as to seal the latter imperviously;
the packaging comprising a screen (20, 22; 24, 26; 26) against electron radiation (E), placed along the cover sheet (14) on the inside of the tub (2) and dimensioned so as to extend above the products (10) to bc sterilized and so as to delimit on the cover sheet (14) a peripheral zone (14a) for fixing this cover sheet (14) to the tub (2).

2. Packaging according to Claim 1, **characterized in that** the screen (20, 22; 26) is attached to the cover sheet (14), in particular by bonding or welding.

3. Packaging according to Claim 1, **characterized in that** the screen (24) is arranged over the products (10) inside the tub (2) prior to the sealing of the latter.

4. Packaging according to Claim 1, **characterized in that** the screen (20, 22; 24, 26) is formed of at least two sheets or layers and **in that** at least one of the said sheets or layers (20; 26) is attached to the cover sheet (14), whereas at least one other of the said sheets or layers (22; 24) is arranged inside the tub (2) prior to the sealing of the latter, either over the products (10) or on ledges provided for that purpose or on a product-positioning piece placed in this tub.

5. Packaging according to one of Claims 1 to 4, **characterized in that** the screen (24; 26) is a metallized or metallic sheet (26).

6. Packaging according to one of Claims 1 to 4, **characterized in that** the screen comprises a plastic material.

7. Packaging according to one of Claims 1 to 4, **characterized in that** the screen comprises a material based on natural fibres, for example plant fibres.

8. Packaging according to one of Claims 1 to 4, **characterized in that** the screen comprises at least two complementary sheets (20, 22) of selectively impervious material.

9. Packaging according to any one of Claims 1 to 8, **characterized in that** the selectively impervious material is a material based on filaments of HDPE or some other polymer, bound together by heat and pressure.

10. Packaging according to any one of Claims 1 to 8, **characterized in that** the selectively impervious material is a material based on plant fibres, for example paper.

11. Method for producing a packaging according to one of Claims 1 to 10, consisting in:
- using a tub (2) made of plastic and a cover sheet (14) made of selectively impervious material, impervious to contamination by micro-organisms, bacteria and/or a biologically active material while at the same time remaining permeable to a sterilization gas;
- sealing the tub (2) imperviously by fixing the peripheral zone (14a) of the cover sheet (14) to the tub (2);
- using a screen (20, 22; 26, 24; 26) against electron radiation (E),
- choosing the shape and dimensions of this screen (20, 22; 26, 24; 26) in such a way that this screen (20, 22; 26, 24; 26) extends, when in the tub (2), above the products (10) to be sterilized and delimits, on the cover sheet (14), the said peripheral zone (14a).

12. Method according to Claim 11, **characterized in that** it comprises the step which consists in attaching the screen (20, 22; 26) to the cover sheet (14), in particular by bonding or welding.

13. Method according to Claim 11, **characterized in that** it comprises the step which consists in arranging the screen (24) over the products (10) inside the tub (2), prior to the sealing of the latter.

14. Method according to Claim 11, **characterized in that** it comprises the steps consisting in:
- using a screen (20, 22; 24, 26) formed by at least two sheets or layers, and
- attaching at least one of the said sheets or layers (20; 26) to the cover sheet (14) and in arranging at least one other of the said sheets or layers (22; 24) inside the tub (2) prior to the sealing of the latter, either over the products (10) or on ledges provided for this purpose, or on a product-positioning piece placed in this tub.

## Patentansprüche

1. Verpackung für sterile Gegenstände oder solche, die durch ein Gas sterilisiert werden sollen, beispielsweise einem Gas vom Typ ETO, mit einer Schachtel (2) aus Kunststoff und einer Abdeckfolie (14) aus einem selektiv dichten Material, das gegenüber der Kontamination durch Mikroorganismen, Bakterien und/oder ein biologisch aktives Material dicht ist, das dabei gegenüber einem Sterilisiergas durchlässig bleibt, und die auf der Schachtel (2) derart befestigt ist, dass sie Letztere dicht verschließt;
wobei die Verpackung eine Abschinnung (20, 22; 24, 26; 26) gegenüber einer Elektroneneinstrahlung (E) aufweist, die entlang der Abdeckfolie (14) auf der Innenseite der Schachtel (2) angeordnet und derart dimensioniert ist, dass sie sich oberhalb der zu sterilisierenden Gegenstände (10) erstreckt und auf der Abdeckfolie (14) eine Umfangszone (14a) der Befestigung dieser Abdeckfolie (14) an der Schachtel (2) abgrenzt.

2. Verpackung nach Anspruch 1, **dadurch gekennzeichnet, dass** die Abschirmung (20, 22; 26) an der Abdeckfolie (14) angebracht ist, insbesondere durch Kleben oder Schweißen.

3. Verpackung nach Anspruch 1, **dadurch gekennzeichnet, dass** die Abschirmung (24) auf den Gegenständen (10) im Innern der Schachtel (2) vor dem Verschließen derselben angeordnet ist.

4. Verpackung nach Anspruch 1, **dadurch gekennzeichnet, dass** die Abschirmung (20, 22; 24, 26) durch zumindest zwei Folien oder Lagen gebildet ist, und dass zumindest eine der Folien oder Lagen (20; 26) an der Abdeckfolie (14) angebracht ist, während zumindest eine weitere der Folien oder Lagen (22; 24) im Inneren der Schachtel (2) vor dem Verschließen derselben angeordnet ist, entweder auf den Gegenständen (10), auf zu diesem Zweck vorgesehenen Auflagen, oder auf einem Pusitionierstück für die Gegenstände, das in der Schachtel angeordnet ist.

5. Verpackung nach einem der Ansprüche 1 bis 4, **dadurch gekennzeichnet, dass** die Abschirmung (24; 26) eine metallisierte oder metallische Folie (26) aufweist.

6. Verpackung nach einem der Ansprüche 1 bis 4, **dadurch gekennzeichnet, dass** die Abschirmung einen Kunststoff aufweist.

7. Verpackung nach einem der Ansprüche 1 bis 4, **dadurch gekennzeichnet, dass** die Abschirmung ein Material auf der Basis von Naturfasern, beispielsweise pflanzlichen Fasern, aufweist.

8. Verpackung nach einem der Ansprüche 1 bis 4, **dadurch gekennzeichnet, dass** die Abschirmung zumindest zwei komplementäre Folien (20, 22) aus selektiv dichtem Material aufweist.

9. Verpackung nach einem der Ansprüche 1 bis 8, **dadurch gekennzeichnet, dass** das selektiv dichte Material ein Material auf der Basis von Filamenten aus PEHD oder einem anderen Polymer ist, die mittels Wärme oder Druck untereinander verbunden sind.

10. Verpackung nach einem der Ansprüche 1 bis 8, **dadurch gekennzeichnet, dass** das selektiv dichte Material ein Material auf der Basis von pflanzlichen Fasem, beispielsweise Papier, ist.

11. Verfahren zur Herstellung einer Verpackung nach einem der Ansprüche 1 bis 10, mit den Schritten:
- Verwenden einer Schachtel (2) aus Kunststoffmaterial und einer Abdeckfolie (14) aus selektiv dichtem Material, das gegenüber der Kontamination durch Mikroorganismen, Bakterien und/oder ein biologisch aktives Material dicht ist, das dabei gegenüber einem Sterilisiergas durchlässig bleibt;
- dichtes Verschließen der Schachtel (2) durch Befestigen der Umfangszone (14a) der Abdeckfolie (14) an der Schachtel (2);
- Verwenden einer Abschirmung (20, 22; 26, 24; 26) gegenüber einer elektronischen Einstrahlung (E),
- Wählen der Form und der Abmessungen dieser Abschirmung (20, 22; 26, 24; 26) derart, dass die Abschirmung (20, 22; 26, 24; 26) sich, wenn sie in der Schachtel (2) ist, über den zu sterilisierenden Gegenständen (10) erstreckt und an der Abdeckfolie (14) die Umfangszone (14a) abgrenzt.

12. Verfahren nach Anspruch 11, **dadurch gekennzeichnet, dass** es den Schritt Anbringen der Abschirmung (20, 22; 26) an der Abdeckfolie (14), insbesondere durch Kleben oder Schweißen, umfasst.

13. Verfahren nach Anspruch 11, **dadurch gekennzeichnet, dass** es den Schritt Anordnen der Abschirmung (24) auf den Gegenständen (10) im Innern der Schachtel (2) vor dem Verschließen derselben umfasst.

14. Verfahren nach Anspruch 11, **dadurch gekennzeichnet, dass** es die Schritte umfasst:
- Verwenden einer Abschirmung (20, 22; 24, 26), die durch zumindest zwei Folien oder Lagen gebildet ist, und
- Anbringen zumindest einer der Folien oder Lagen (20; 26) auf der Abdeckfolie (14) und Anordnen zumindest einer weiteren der Folien oder Lagen (22; 24) im Innern der Schachtel (2) vor dem Verschließen derselben, entweder auf den Gegenständen (10), auf zu diesem Zweck vorgesehenen Auflagen oder auf einem Positionierstück für die Gegenstände, das in der Schachtel angeordnet ist.
